# EUROPÄISCHE PATENTANMELDUNG

(11) **EP 3 454 055 A2**
(43) Veröffentlichungstag der Anmeldung: **13.03.2019**
(21) Anmeldenummer: 18187816.6
(22) Anmeldetag: 07.08.2018
(51) Int. Cl.: G01N 27/22, G01N 33/00, G01W 1/17, G08B 21/20, F24F 11/30, G01N 33/38

(54) **VORRICHTUNG UND VERFAHREN ZUM MESSEN EINER LUFTFEUCHTIGKEIT AN EINEM MESSORT**

(30) Priorität: 07.08.2017 DE 102017117935
(71) Anmelder: Reime, Gerd, 77815 Bühl (DE)
(72) Erfinder: Reime, Gerd, 77815 Bühl (DE)
(74) Vertreter: RPK Patentanwälte Reinhardt, Pohlmann und Kaufmann Partnerschaft mbB

(57) **Zusammenfassung**

Es wird eine Messanordnung (1) zum Messen einer Luftfeuchtigkeit (3) an einem Messort (5), mit einer elektrischen Energiequelle (7), mittels der die Messanordnung (1) mit elektrischer Energie versorgbar ist, einer Sensorvorrichtung (9), mittels der die Luftfeuchtigkeit (3) messbar ist, einer der Sensorvorrichtung (9) nachgeschalteten Speichervorrichtung (11), mittels der eine von der gemessenen Luftfeuchtigkeit (3) abhängige Feuchtigkeitsgröße (13) speicherbar ist und einer der Speichervorrichtung (11) nachgeschalteten Schnittstellenvorrichtung (15), mittels der die Feuchtigkeitsgröße (13) an eine von der Messanordnung (1) unabhängige Auslesevorrichtung (17) übermittelbar ist vorgeschlagen.

Die Messanordnung (1) ermöglicht auf bequeme Art und Weise eine direkte Rückmeldung/optische Signalisierung, ob und/oder wie lange gelüftet werden muss, bis an dem Messort (5) ein unkritisches Raumklima herrscht und ermöglicht dadurch eine einfache Schimmelüberwachung. Zusätzlich kann, beispielsweise in Intervallen von einem Jahr, die Messanordnung (1) für eine Langzeitbewertung ausgelesen werden um insbesondere das Beachten der direkten Rückmeldungen der Messanordnung (1) durch einen Nutzer beurteilen zu können. Das Auslesen kann berührungsfrei, optisch und/oder über ein Netzwerk erfolgen, insbesondere zur weiteren Verarbeitung der jeweiligen Kurzzeitmessungen. Dazu hat die Messanordnung (1) eine der Speichervorrichtung (11) nachgeschaltete Schnittstellenvorrichtungen (15), mittels der die Feuchtigkeitsgröße (13) an eine von der Messanordnung (1) unabhängige Auslesevorrichtung (17) übermittelbar ist.

## Beschreibung

Die Erfindung betrifft eine Messanordnung zum Messen einer Luftfeuchtigkeit an einem Messort, mit einer Sensorvorrichtung, mittels der die Luftfeuchtigkeit messbar ist, eine mit der Messanordnung zusammenwirkende Vorrichtung, ein die Messanordnung und die Vorrichtung aufweisendes Geräteset sowie ein Vorrichtungsgemäßes Verfahren zum Messen einer Luftfeuchtigkeit an einem Messort.

Das Messen einer Luftfeuchtigkeit an einem Messort dient beispielsweise zum Erkennen oder Vorhersagen eines Raumklimas, beispielsweise als Eingangsgröße zum Beeinflussen, Steuern und/oder Regeln des Raumklimas. Insbesondere kann von Interesse sein, ob die Luftfeuchtigkeit in einem Bereich liegt, in dem eine Neigung zur Schimmelbildung vorliegt oder nicht. Es sind Ausgestaltungen bekannt, bei denen die Luftfeuchtigkeit erfasst und im Sinne eines Indikators angezeigt wird, beispielsweise als Warnung oder als Hinweis eine bestimmte manuelle Maßnahme zum Beeinflussen des Raumklimas zu ergreifen. Entsprechende Vorrichtungen können insbesondere über ein Stromnetz mit elektrischer Energie versorgt werden, um entsprechende Sensoren und Anzeigevorrichtungen zu betreiben. Außerdem sind Geräte bekannt, die unterschiedliche Messwerte erfassen, beispielsweise zusätzlich zu der Luftfeuchtigkeit eine Temperatur. Insbesondere können mehrere Messstellen für Temperatur und Luftfeuchtigkeit vorhanden sein, beispielsweise zum Überwachen des Raumklimas und zusätzlich eines Feuchtegehaltes einer einen entsprechenden Raum begrenzenden Wand.

Aus der DE 10 2014 107 690 A1 ist ein Schimmelwarngerät zur Überwachung des Raumklimas bekannt. Um eine sichere und zuverlässige Vorhersage der Gefahr von Schimmelbildung zu ermöglichen, wird vorgeschlagen, dass das Schimmelwarngerät einen Sensor zur Messung von Raumfeuchtigkeit umfasst, sowie weiterhin einen Sensor zur Messung der Raumtemperatur, einen Sensor zur Messung der Raumfeuchtigkeit und einen Sensor zur Messung der Wandtemperatur, einen mit den Sensoren verbundenen Mikrocontroller, welcher mit einer Software eingerichtet ist, die Sensordaten periodisch abzufragen, zu verarbeiten und zu speichern und mindestens ein Element zur Anzeige von Daten.

Die DE 33 00 389 A1 betrifft ein besonders kleines, einfach zu handhabendes universell einsetzbares Gerät zu Messen der Klimawerte, insbesondere in Wohn-, Schlaf- und Arbeitsräumen. Das Gerät hat Einrichtungen zum Messen der Lufttemperatur und der Luftfeuchtigkeit sowie einen Anschluss an das Stromversorgungsnetz und einen Mikroprozessor, der die gemessene Lufttemperatur und Luftfeuchtigkeitswerte erfasst und Behaglichkeitskoordinaten aus Lufttemperaturen relativer Luftfeuchtigkeit ständig berechnet. Beim Erreichen von Grenzwerten in einem vorprogrammierten Behaglichkeitsfeld leuchten an einem Gehäuse des Gerätes nebeneinander angeordnete elektronische Warnleuchten auf. Das Gerät kann zum Warnen vor einem Unterschreiten oder Überschreiten der Grenzwerte, für Langzeitmessungen und zur automatischen Steuerung von Heizungsventilen und Lüftungseinrichtungen eingesetzt werden.

### Darstellung der Erfindung

Aufgabe der Erfindung ist es, eine vereinfachte und umfassende Überwachung eines Raumklimas zu ermöglichen, insbesondere für eine Vielzahl von Räumen mit einer Eignung für kurzfristige Messungen bei einer gleichzeitig gegebenen Möglichkeit einer Langzeitüberwachung des Raumklimas, insbesondere mit einer vereinfachten Energieversorgung.

Die Aufgabe wird bei einer Messanordnung zum Messen einer Luftfeuchtigkeit an einem Messort, mit einer elektrischen Energiequelle, mittels der die Messanordnung mit elektrischer Energie versorgbar ist, einer Sensorvorrichtung, mittels der die Luftfeuchtigkeit messbar ist und einer der Sensorvorrichtung nachgeschalteten Speichervorrichtung, mittels der eine von der gemessenen Luftfeuchtigkeit abhängige Feuchtigkeitsgröße speicherbar ist, durch eine der Speichervorrichtung nachgeschaltete Schnittstellenvorrichtung, mittels der die Feuchtigkeitsgröße an eine von der Messanordnung unabhängige Auslesevorrichtung übermittelbar ist, gelöst. Dadurch ist es möglich, die Messanordnung für einen bestimmten Zeitraum zu betreiben, insbesondere eine Langzeitmessung durchzuführen, und das Ergebnis der Messung mittels der von der Messanordnung unabhängigen Auslesevorrichtung auszulesen. Dies ermöglicht auch, dass eine Vielzahl der Messanordnungen in einem Raum oder einer Vielzahl von Räumen positioniert werden können, wobei entsprechende Messergebnisse der Vielzahl von Messanordnungen mit ein und derselben Auslesevorrichtung abgefragt bzw. ausgelesen werden können. Die Auslesevorrichtung ist also dazu eingerichtet, ausgelegt und/oder konstruiert um mit einer Vielzahl von einzelnen Messanordnungen, insbesondere zur Überwachung einer Vielzahl von Messanordnungen und/oder Räumen, und/oder Messorten, zusammenzuwirken, insbesondere um die einzelnen Messanordnungen nacheinander abzufragen bzw. deren Messergebnisse auszulesen. Dadurch können die einzelnen Messanordnungen vergleichsweise klein und günstig realisiert werden, sodass sich zum Überwachen von einer Vielzahl von Messanordnungen ein vergleichsweise einfach aufgebautes System ergibt, wobei vorzugsweise nur eine einzige Auslesevorrichtung zum Abfragen bzw. Erfassen der Vielzahl von Messergebnissen benötigt wird. Räume können damit vollständig und vereinfacht überwacht werden, beispielsweise um schnell und von Jedermann durchführbar Erkenntnisse über eine Bauphysik einer Gebäudehülle zu erhalten. Es kann ohne tiefere Kenntnisse hinsichtlich Bauphysik wie Dämmung, Feuchtespeicherfähigkeit und/oder Dampfdurchlässigkeit ermittelt werden, ob ein kritischer Zustand vorliegt oder nicht. Insbesondere können Wärmebrücken erkannt und bewertet werden, beispielweise ob diese kritische Feuchtigkeitswerte verursachen. Dazu können beispielsweise mehrere Messorte gemessen und miteinander verglichen und/oder vermeintlich kritische Stellen wie Ecken, Leibungen, etc. als Messorte ausgewählt werden. Die Feuchtigkeitsgröße kann alternativ oder zusätzlich auch eine Temperaturgröße aufweisen und/oder zusätzlich von einer Temperatur an dem Messort abhängen oder davon abhängig berechnet sein. Außerdem kann die Feuchtigkeitsgröße zusätzlich eine Kennung zum Identifizieren der Messanordnung und/oder einen Zeitstempel eines Messzeitpunktes oder Messzeitraums aufweisen. Die Messanordnung kann vorzugsweise auf bequeme Art und Weise eine direkte Rückmeldung ermöglichen, beispielsweise durch eine optische Signalisierung, ob und/oder wie lange gelüftet werden muss, bis an dem Messort ein unkritisches Raumklima herrscht. Dadurch wird eine einfache Schimmelüberwachung ermöglicht. Zusätzlich kann, beispielsweise in Intervallen von einem Jahr, die Messanordnung für eine Langzeitbewertung ausgelesen werden um insbesondere das Beachten der direkten Rückmeldungen der Messanordnung durch einen Nutzer beurteilen zu können. Das Auslesen kann berührungsfrei, optisch und/oder über ein Netzwerk erfolgen, insbesondere zur weiteren Verarbeitung der jeweiligen Kurzzeitmessungen.

Bei einem bevorzugten Ausführungsbeispiel der Messanordnung ist vorgesehen, dass mittels der elektrischen Energiequelle elektrische Pulse erzeugbar sind, wobei die Messanordnung eine Diodenvorrichtung aufweist, mittels der die elektrischen Pulse spannungsbegrenzbar sind und dabei gleichzeitig ein von der Luftfeuchtigkeit abhängiges vorzugsweise optisches Signal anzeigbar ist. Die Messanordnung kann insbesondere dazu ausgelegt sein, die Luftfeuchtigkeit und/oder eine Temperatur zyklisch, also über der Zeit, insbesondere zyklisch über einer Vielzahl von Zeitpunkten zu messen. Dazu kann insbesondere eine von der Luftfeuchtigkeit und/oder der Temperatur abhängige Messgröße ermittelt werden bzw. ermittelbar sein. Die Diodenvorrichtung kann zumindest eine Leuchtdiode, vorzugsweise zwei Leuchtdioden aufweisen. Bei den elektrischen Pulsen kann es sich vorzugsweise um Rückschlagimpulse einer Induktivität handeln. Mittels der elektrischen Pulse kann für jede einzelne der Messungen die Messanordnung mit elektrischer Energie versorgt werden. Dazwischen kann die Messanordnung im Wesentlichen stromlos geschaltet sein, sodass sich in Summe ein sehr geringer Stromverbrauch ergibt. In den als im Wesentlichen stromlos bezeichneten Wartezeiten kann ein Stromverbrauch von kleiner 200 Nanoampere, insbesondere kleiner 100 Nanoampere, bevorzugt zwischen 20 und 100 Nanoampere auftreten. Dieser Stromfluss dient beispielsweise zur Versorgung einer Pulsgeneratorvorrichtung. Die eigentliche Messung kann beispielsweise alle 3 Sekunden stattfinden. Im Durchschnitt ist dann ein Gesamtstromverbrauch von kleiner als 5 Mikroampere bei beispielsweise 3 Volt erzielbar. Die elektrische Energiequelle zur Versorgung der Messanordnung muss also für die Dauer des Messzeitraums eine Leistung von kleiner gleich 15 Mikrowatt aufbringen. Diese geringe Dauerleistung beinhaltet vorzugsweise nicht nur die Messung sondern zusätzlich auch die regelmäßige Bereitstellung an der Schnittstellenvorrichtung und/oder die regelmäßige Signalisierung, die beispielsweise mittels Leuchtdioden erfolgen kann.

Die elektrische Energiequelle kann ein Pulsgenerator zum Erzeugen der elektrischen Pulse aufweisen. Grundsätzlich ist es wünschenswert, dass für eine besonders genaue Messung eine konstante Spannung zur Versorgung der Sensorvorrichtung vorliegt. Unabhängig von einer Ausgangsspannung des Pulsgenerators, also falls dieser gegebenenfalls über der Zeit Pulse mit veränderlichen Spannungen erzeugt, beispielsweise aufgrund einer entsprechenden schwankenden Energieversorgung des Pulsgenerators, ist es dank der Diodenvorrichtung möglich, die elektrischen Pulse in der Spannung zu begrenzen und damit eine konstante Spannung der elektrischen Pulse für eine besonders genaue Messung der Luftfeuchtigkeit zu erzeugen bzw. bereitzustellen. Optional ist es denkbar, dass die Diodenvorrichtung nur zum Regeln der Spannung dient und dazu beispielsweis eine Zenerdiode aufweist. Bevorzugt kann jedoch die Diodenvorrichtung zumindest eine Leuchtdiode aufweisen, wobei diese aufgrund Ihrer stark nicht linearen Kennlinie ebenfalls zur Spannungsbegrenzung und damit zur Regelung der Spannung der elektrischen Pulse verwendet werden kann wobei gleichzeitig das Anzeigen des Signals erfolgen kann, insbesondere in Form eines Blitzsignals, insbesondere eines Blitzsignals für die Dauer des entsprechenden elektrischen Impulses. Mittels einer Messvorrichtung kann eine Messgröße ermittelbar sein, wobei diese insbesondere in Form einer elektrischen Spannung und/oder eines elektrischen Stromes an die nachgeschaltete Diodenvorrichtung weitergeleitet werden kann, sodass diese anzeigbar ist. Die Diodenvorrichtung kann insbesondere zwei Dioden aufweisen, insbesondere so geschaltet sein, dass jeweils eine der Dioden aufleuchtet falls die Luftfeuchtigkeit einen vorgegebenen oder vorgebbaren Wert unter- oder überschreitet. Die Messanordnung ist jederzeit ablesbar, beispielsweise durch eine Person, die den zu überwachenden Raum häufig betritt. Diese kann z.B. zu Nachweiszwecke eine manuelle Protokollierung durchführen. Außerdem kann z.B. durch eine mit der Überwachung des Raumklimas beauftragte Person die Auslesung vornehmen und insbesondere anhand der Feuchtigkeitsgröße sehr einfach eine Aussage auch über die Bauphysik der Raumhülle treffen. Es ist also ein einfacher und doppelter Nachweis des Raumklimas möglich. Die Messanordnung ist sehr günstig, wegen einer geringen Teilekomplexität kompakt aufbaubar und verbraucht sehr wenig Strom. Für entsprechende Messungen kann diese unauffällig an kritischen Stellen, beispielsweise in Ecken positioniert, dort direkt manuell abgelesen und nach erfolgter Langzeitmessung mittels der Auslesevorrichtung ausgelesen werden. Außerdem kann abgelesen werden, ob ein Fenster zu öffnen ist oder wieder geschlossen werden kann. Beispielsweise durch eine Widergabe eines optischen roten Signals für Öffnen und eines grünen Signals falls das Fenster wieder geschlossen werden kann, beispielsweise nach dem Entfernen eines starken Feuchteeintrags durch Kochen oder Duschen durch Fensterlüften.

Außerdem ist es bevorzugt möglich, dass die elektrische Energiequelle einen Wandler zum Wandeln einer in einer Umgebung der Messanordnung vorhandenen Umgebungsenergie in elektrische Energie aufweist, mittels dem die Messanordnung Energieautark und/oder netzunabhängig mit elektrischer Energie versorgbar ist und/oder einen Energiespeicher aufweist, mittels der die Messanordnung für einen Zeitraum von insbesondere >1 Jahr, >2 Jahre, >5 Jahre, besonders bevorzugt >10 Jahre mit elektrischer Energie versorgbar ist. Es ist also eine besonders unkomplizierte und einfache Energieversorgung möglich. Netzanschlüsse und/oder ein Nachladen von Akkumulatoren sind nicht erforderlich. Bei dem Wandler kann es sich um eine Fotozelle handeln, die in der Umgebung vorhandenes Licht in elektrische Energie umwandelt. Alternativ oder zusätzlich kann der Wandler auch als thermoelektrischer Wandler ausgestaltet sein mittels dem in der Umgebung vorhandene Wärme in elektrische Energie wandelbar ist. Bei dem alternativen oder zusätzlich vorhandenen Energiespeicher kann es sich um einen Pufferspeicher, beispielsweise einen Kondensator zum Zwischenspeichern der von dem Wandler erzeugten elektrischen Energie handeln. Außerdem ist es denkbar, dass der Energiespeicher in Form einer Batterie ausgebildet ist, der für die oben genannten Zeiträume einen ausreichenden Energievorrat beinhaltet bzw. zur Verfügung stellen kann. Unter energieautark und/oder netzunabhängig elektrisch versorgbar kann verstanden werden, dass die Messanordnung unbegrenzt oder zumindest für die oben angegebenen Zeiträume ohne weiteres Zuführen von elektrischer Energie und/oder wechseln von Batterien und/oder Nachladen von Akkumulatoren betreibbar ist, insbesondere unterbrechungsfrei, beziehungsweise in einem gepulsten Dauerbetrieb betreibbar ist, insbesondere zum Durchführen einer Langzeitmessung. Es kann also über Zeiträume von größer als einem Jahr eine Langzeitmessung der Luftfeuchtigkeit erfolgen, ohne dass ein durch die Messanordnung entsprechend überwachter Raum für Wartungsarbeiten wie nachladen und/oder wechseln von Akkumulatoren betreten werden muss. Für entsprechende Langzeitmessungen wie beispielsweise ein Erfassen von Verbrauchsdaten ohnehin vorgesehenes Wartungsintervall beispielsweise von circa einem Jahr, kann mitgenutzt werden zum Erfassen eines Ergebnisses einer entsprechenden mittels der Messanordnung durchgeführten Langzeitmessung de Luftfeuchtigkeit.

Bei einer weiteren Ausgestaltung ist vorgesehen, dass mittels der Messanordnung eine von der Luftfeuchtigkeit abhängige binäre Schwellwertgröße erzeugbar ist. Unter einer Schwellwertgröße kann eine Größe verstanden werden, die zumindest zwei Werte annimmt, insbesondere einen ersten Wert falls die Luftfeuchtigkeit einen vorgegebenen Schwellwert unterschreitet und einen zweiten Wert falls die Luftfeuchtigkeit den vorgegebenen oder vorgebbaren Schwellwert überschreitet. Es ist möglich, dass die Schwellwertgröße weiterverarbeitet und/oder ausgelesen und/oder gespeichert wird. Insbesondere kann die Schwellwertgröße zur Ansteuerung der Diodenvorrichtung verwendet werden, Die Diodenvorrichtung kann zumindest eine Leuchtdiode aufweisen. Insbesondere kann für den ersten Wert der Schwellwertgröße ein erstes Signal und für den zweiten Wert der Schwellwertgröße ein zweites Signal wiedergegeben werden, insbesondere in Form eines Blitzsignals erzeugt durch unterschiedliche Leuchtdioden der Diodenvorrichtung. Das Vorgeben des Schwellwertes kann insbesondere durch einen Vergleich einer Messstrecke zum Erfassen der Luftfeuchtigkeit mit einer Referenzstrecke erfolgen. Als Ausgang eines entsprechenden Vergleichs liegt im Sinne einer Messgröße die Schwellwertgröße vor. Wie vorab beschrieben, kann die Schwellwertgröße zyklisch, insbesondere jeweils für die Dauer eines der elektrischen Pulse in Abhängigkeit von der Feuchtigkeit und/oder Temperatur ermittelt werden. Die Messgröße kann also für jeden der elektrischen Pulse, insbesondere erzeugt oder erzeugbar mittels einer Pulserzeugungsvorrichtung der Messanordnung, einmalig ermittelbar, anzeigbar, speicherbar und/oder mit der Feuchtigkeitsgröße verrechenbar sein. Unter verrechenbar kann verstanden werden, dass die jeweils ermittelte Messgröße, insbesondere die Schwellwertgröße pro Zyklus, also pro elektrischem Puls als Eingangsgröße für einen Rechenalgorithmus zum Bestimmen der Feuchtigkeitsgröße verwendet wird. Insbesondere kann eine Länge bestimmt werden, für die ein vorgegebener oder vorgebbarer Schwellwert für die Luftfeuchtigkeit überschritten ist. Alternativ oder zusätzlich kann eine durchschnittliche Überschreitungsdauer einzelner Überschreitungen des Schwellwertes während einer Langzeitmessung ermittelt, bereitgestellt und/oder ausgegeben werden. Die Feuchtigkeitsgröße erlaubt einen Aufschluss darüber, ob es in dem überwachten Raum tendenziell zu feucht oder gegebenenfalls trocken war. Alternativ oder zusätzlich kann die Messung in einem oder einer Vielzahl von Intervallen durchgeführt werden, die so lange sind, wie eine Bildung von Schimmel mindestens dauert. Falls in einem solchen Intervall die Feuchtigkeitsgröße eine weitere Schwelle überschreitet, kann auf eine erhöhte Schimmelbildungsgefahr geschlossen werden. Die Feuchtigkeitsgröße kann ein Verhältnis von Zuständen, die in Ordnung sind und nicht in Ordnung sind aufweisen. Dadurch ist vorzugsweise für das jeweilige Intervall oder die Zeitdauer der Messung eine Aussage möglich, ob ausreichend Maßnahmen zur Reduzierung der Feuchtigkeit ergriffen wurden oder nicht. Das Verhältnis kann beispielsweise in Prozent bezogen auf die Messdauer angegeben werden.

Außerdem kann die Messanordnung so aufgebaut sein, dass die Speichervorrichtung Teil einer Datenerfassung ist, die abhängig von der Luftfeuchtigkeit und/oder Temperatur zyklisch die Feuchtigkeitsgröße berechnet, ermittelt und/oder erfasst. Bei der Datenerfassung kann es sich in einer einfachen Ausgestaltung um einen Addierer handeln, der die Zeiten korrekten Lüftens und/oder unkorrekten Lüftens aufaddiert und/oder zu der Feuchtigkeitsgröße verrechnet. Es erfolgt also bereits in der Messanordnung eine Verarbeitung der zyklisch anfallenden Messgröße und vorzugsweise damit eine Bewertung der Luftfeuchtigkeit über einen längeren Zeitraum hinweg.

Alternativ oder zusätzlich weist die Messanordnung eine von der Luftfeuchtigkeit des Messorts beeinflussbare Messstrecke und eine definierte oder justierbare elektrische Eigenschaften aufweisende Referenzstrecke auf, denen ein Komparator nachgeschaltet ist.

Die Referenzstrecke und die Messstrecke können so aufeinander abgestimmt sein, dass diese bei einer bestimmten Luftfeuchtigkeit identische elektrische Eigenschaften aufweisen, insbesondere falls diese gleichermaßen mit einem der elektrischen Pulse bestromt werden, eine identische Ausgangsspannung liefern. Die Ausgangsspannungen werden mittels des Komparators miteinander verglichen, sodass im Ergebnis als Ausgangsgröße des Komparators die Messgröße, insbesondere in Form der vorab beschriebenen Schwellwertgröße, erzeugt wird bzw. erzeugbar ist. Als Ausgangsgröße des Komparators kann also eine binäre Messgröße erzeugt werden. Insbesondere ist es möglich, dass die Diodenvorrichtung und/oder die Speichervorrichtung dem Komparator nachgeschaltet ist/sind, vorzugsweise direkt nachgeschaltet sind. Damit ist es möglich, dass der Komparator die Anzeige steuert und/oder mit elektrischer Energie versorgt, insbesondere die zum Erzeugen des Signals notwendige Energie liefert. Bevorzugt weist die Referenzstrecke einen eine Kapazität aufweisenden Feuchtigkeitssensor auf. Es sind Feuchtigkeitssensoren bekannt, die abhängig von einer Feuchtigkeit aufquellen oder schrumpfen, insbesondere zum Beeinflussen der Kapazität. Dementsprechend weist auch die Referenzstrecke einen Kondensator auf, der entsprechend des vorgegebenen oder vorgebbaren Schwellwertes an den Feuchtigkeitssensor angepasst ist. Alternativ oder zusätzlich kann der Kondensator der Referenzstrecke zum Beeinflussen des Schwellwertes justierbar bzw. einstellbar sein. Grundsätzlich sind verschiedenartigste Feuchtigkeitssensoren denkbar, wobei je nach elektrischen Eigenschaften die Referenzstrecke entsprechend aufgebaut bzw. an den entsprechenden Feuchtigkeitssensor hinsichtlich der elektrischen Eigenschaften angepasst ist. Darüber hinaus ist es denkbar, dass die Messvorrichtung einen integrierten Schaltkreis aufweist, der digitale Informationen liefert, beispielsweise Temperaturwerte und/oder Feuchtigkeitswerte.

Gemäß einer weiteren bevorzugten Alternative können die Messstrecke und/oder die Referenzstrecke eine Temperaturkompensation und/oder Temperaturmessstelle aufweisen. Die Temperaturkompensation ermöglicht eine zusätzliche Bewertung eines Temperatureinflusses. Beispielsweise kann ein von einer Temperatur abhängiges Wachstum von Schimmel und/oder der Umstand, dass die Feuchtigkeitsaufnahme von Wasser in Luft von der Temperatur abhängig ist berücksichtigt werden. Die Temperaturkompensation kann beispielsweise einen temperaturabhängigen Widerstand, beispielsweise als NTC oder PTC Widerstand ausgeführt aufweisen. Ein solcher Widerstand kann in Serie oder in einem Parallelzweig in die Messstrecke und/oder in die Referenzstrecke geschaltet werden. Dadurch beeinflusst also auch die an dem Messort herrschende Temperatur die Messgröße, insbesondere die Schwellwertgröße. Die Messgröße bzw. Schwellwertgröße ist damit also von der Luftfeuchtigkeit abhängig und/oder von einer Temperatur am Messort.

Zum Schützen vor unerwünschten Einwirkungen kann die Messanordnung eine Manipulationsschutzvorrichtung aufweisen, mittels der die Messanordnung ortsfest an dem Messort fixierbar, verschließbar und/oder zumindest ein Entfernen von dem Messort und/oder ein Öffnen anzeigbar sind. Unter einer Manipulationsschutzvorrichtung kann insbesondere eine Plombe, ein sich bei einem Öffnen selbst zerstörender Verschluss und/oder eine Kennzeichnung, die ein Öffnen, Abmontieren, Entfernen und/oder ähnliches anzeigt, verstanden werden. Durch die Manipulationsschutzvorrichtung kann die Messanordnung auch in Räumen betrieben werden, die von an der Messung nicht beteiligten Personen betreten werden können. Für den Fall, dass die Manipulationsschutzvorrichtung noch in Takt ist, kann mit einer hohen Wahrscheinlichkeit davon ausgegangen werden, dass keine Manipulation vorliegt, also eine entsprechende Messung der Luftfeuchtigkeit verlässliche Werte liefert.

Die Aufgabe ist außerdem durch eine Auslesevorrichtung zum Auslesen einer in einer vorab beschriebenen Messanordnung gespeicherten Feuchtigkeitsgröße gelöst, wobei die Auslesevorrichtung mit einer Ausleseschnittstellenvorrichtung versehen ist. Über die Ausleseschnittstellenvorrichtung können zum Auslesen der Feuchtigkeitsgröße Daten von der Schnittstellenvorrichtung der Messanordnung übertragen werden. Die Ausleseschnittstellenvorrichtung wirkt also zum Auslesen der Feuchtigkeitsgröße mit der Schnittstellenvorrichtung der Messanordnung zusammen. Dadurch kann zwischen der Auslesevorrichtung und der Messanordnung eine Datenstrecke aufgebaut werden, beispielsweise optisch und/oder über Funk, beispielsweise über ein Opto-Id-Verfahren, bei dem eine optische bidirektionale Kommunikation über die Datenstrecke läuft. Insbesondere kann die optische Verbindung über Distanzen von > 1 m, vorzugsweise bis zu 5 m, insbesondere bis zu 10 m aufgebaut werden. Die Datenstrecke muss besonders bevorzugt nur zum eigentlichen Übertragen der Feuchtigkeitsgröße etabliert werden. Dazu kann die Auslesevorrichtung ein Signal, insbesondere optisches und/oder Funksignal, an die Messanordnung senden, um diese für die Datenübertragung aufzuwecken. Alternativ ist es auch denkbar, dass pro Zyklus oder in einem bestimmten Zeitintervall, beispielsweise nur bei jedem 10. Zyklus die Feuchtigkeitsgröße unaufgefordert gesendet wird, insbesondere falls in ein Netzwerk gespeist wird, vorzugsweise über Funk wie WLAN. Die insbesondere zyklisch und/oder unaufgefordert gesendete Feuchtigkeitsgröße ist von der Auslesevorrichtung empfangbar. Dadurch kann Energie eingespart werden. Während des Durchführens der Messung der Luftfeuchtigkeit ist also das Aufbauen bzw. das Aufrechterhalten der Datenstrecke nicht erforderlich. Dadurch kann ein apparativer Aufwand eingespart und ein Energieverbrauch gesenkt werden. Die Auslesevorrichtung kann als mobiles tragbares Gerät aufgebaut sein. Insbesondere kann es sich bei der Auslesevorrichtung um ein universell programmierbares Gerät handeln, das zum Kommunizieren mit der Messanordnung softwaretechnisch eingerichtet, ausgelegt und/oder konstruiert ist. Bei der Verwendung einer Vielzahl von Messanordnungen 1 können diese jeweils eine Kennung beziehungsweise Adresse aufweisen, die beim Auslesen zur Kennzeichnung des jeweiligen Messortes der jeweils ausgelesenen Feuchtigkeitsgröße dienen kann. Ferner kann jeweils ein Zeitstempel mitübertragen werden.

Die Aufgabe ist ferner durch ein Verfahren zum Messen, speichern und/oder auslesen einer Luftfeuchtigkeit an einem Messort mittels einer Messanordnung, insbesondere einer vorab beschriebenen Messanordnung, durch Messen der Luftfeuchtigkeit an dem Messort für eine Vielzahl einzelner Zeitpunkte, Ermitteln für jeden der Zeitpunkte jeweils eine von der jeweils gemessenen Luftfeuchtigkeit abhängige Messgröße, Ermitteln einer von den einzelnen Messgrößen abhängigen Feuchtigkeitsgröße und Bereitstellen der Feuchtigkeitsgröße gelöst. Bei der Messgröße kann es sich insbesondere um eine Schwellwertgröße handeln. Die Feuchtigkeitsgröße wird insbesondere aus den zyklisch vorliegenden einzelnen Messgrößen berechnet, insbesondere durch addieren und subtrahieren, insbesondere durch integrieren, insbesondere durch binäres integrieren. Gemäß einer Alternative kann die Feuchtigkeitsgröße auch als Feld, das die einzelnen Messgrößen enthält, vorliegen. Die so ermittelte bzw. errechnete Feuchtigkeitsgröße kann besonders einfach auf die von der Messanordnung unabhängige Auslesevorrichtung übertragen werden. Vorteilhaft findet vorzugsweise bereits in der Messanordnung eine Interpretation der zyklisch zu den Zeitpunkten vorliegenden Messgrößen statt. Es muss dann lediglich das Ergebnis an die Auslesevorrichtung übertragen werden. Dadurch kann also sofort nach dem Übertragen das Ergebnis einer etwaigen Langzeitmessung mittels der Messanordnung vorliegen, insbesondere von der Auslesevorrichtung direkt an einer entsprechenden Stelle gespeichert und/oder angezeigt werden. Dadurch ist es auf besonders einfache Art und Weise möglich eine Vielzahl von Messorten hinsichtlich der Luftfeuchtigkeit zu überwachen und/oder über einen längeren Zeitraum dort die Luftfeuchtigkeit zu messen und so wie die Messwerte pro Zyklus anfallen diese an den Messort bzw. in der Messanordnung zu verarbeiten und in Form des Ergebnisses an der Schnittstelle für die Auslesevorrichtung bereitzustellen. Die Feuchtigkeitsgröße wird bereitgestellt, insbesondere zur Übermittlung an die Auslesevorrichtung und/oder vorzugsweise nur zum Anzeigen.

Eine bevorzugte Ausführungsform des Verfahrens wird mit Ermitteln der Messgrößen jeweils als binäre Schwellwertgröße, die einen ersten Wert und einen zweiten Wert annehmen kann und Ermitteln der Feuchtigkeitsgröße jeweils durch Erhöhen und/oder erniedrigen in Abhängigkeit des ersten Wertes und/oder zweiten Wertes durchgeführt. Zwischen den entsprechenden Messzyklen, insbesondere zwischen den elektrischen Pulsen kann die bis zu diesem Zeitpunkt errechnete Feuchtigkeitsgröße zwischengespeichert werden. Sobald an dem nächsten Zeitpunkt ein neuer Messwert vorliegt, erfolgt das Verrechnen, also das Erhöhen und/oder Erniedrigen der Feuchtigkeitsgröße und erneut ein entsprechendes Zwischenspeichern, insbesondere so lange bis die Feuchtigkeitsgröße mittels der Auslesevorrichtung ausgelesen wird. Alternativ oder zusätzlich ist es denkbar, dass nach einem Etablieren und erfolgreichen auslesen der Feuchtigkeitsgröße diese auf einen Initialwert zurückgesetzt wird, insbesondere auf null gesetzt wird. Dadurch kann eine neue Messung stattfinden. Alternativ ist es jedoch auch denkbar, den aktuell bei dem Auslesen vorhandenen Wert der Feuchtigkeitsgröße unverändert zu lassen, also diesen für eine Fortsetzung der Langzeitmessung unverändert zu lassen. Die an dem jeweiligen Zeitpunkt fortgeschriebene und gespeicherte, also bereitgestellte Feuchtigkeitsgröße kann mittels der Auslesevorrichtung ausgelesen werden. Dazu wirkt diese mit der Messanordnung zusammen.

Gemäß einer weiteren Alternative erfolgt bevorzugt ein manipulationssicheres und/oder manipulationsanzeigendes Verschließen und/oder Fixieren der Messordnung an dem Messort während des Messens der Luftfeuchtigkeit. Gerade für Langzeitmessungen ist es erforderlich, dass zu einem späteren Zeitpunkt des Auslesens Sicherheit darüber herrscht, dass ein Ergebnis der Langzeitmessung, also die Feuchtigkeitsgröße nicht manipuliert wurde. Dazu ist es erforderlich, dass die Messanordnung während der Messung nicht von dem Messort entfernt wurde und/oder während der Messung nicht geöffnet und nicht manipuliert wurde. Falls dennoch eine Manipulation stattgefunden hat, ist es zumindest vorteilhaft dies sofort zu erkennen. Insofern kann unter manipulationssicherem Verschließen verstanden werden, dass ein entsprechender Verschluss nicht wieder geschlossen werden kann und/oder bei einem nicht autorisierten Öffnen zerstört oder zumindest erkennbar verändert wird. Sofern danach ein erneutes Verschließen erfolgt, wird zumindest das nicht autorisierte Öffnen angezeigt, was vorliegend als manipulationsanzeigendes Verschließen verstanden werden soll.

Gemäß einer bevorzugten Ausführungsform kann das Verfahren schließlich mit Erzeugen eines elektrischen Pulses, Anzeigen der Messgröße, Spannungsbegrenzen des elektrischen Pulses während und durch das Anzeigen der Messgröße sowie Ermitteln der Messgröße während und unter Verwendung der elektrischen Energie des spannungsbegrenzten elektrischen Pulses durchgeführt werden. Dadurch dass gleichzeitig und während des Anzeigens der Messgröße das spannungsbegrenzen des elektrischen Pulses erfolgt, kann einerseits eine möglichst exakte Messung stattfinden, da diese mit einer spannungsbegrenzten, also geregelten Eingangsspannung durchgeführt werden kann. Andererseits können Bauteile an einer entsprechenden Messanordnung eingespart werden, also insbesondere mittels nur eines Bauteils gleichzeitig die Spannungsbegrenzung und das Anzeigen erfolgen.

Das Verfahren wird insbesondere mit der vorab beschriebenen Messanordnung, Auslesevorrichtung und/oder dem vorab beschriebenen Geräteset durchgeführt. Insofern ergeben sich die vorab beschriebenen Vorteile.

Die Aufgabe ist schließlich durch ein Geräteset mit einer vorab beschriebenen Messanordnung und einer vorab beschriebenen Auslesevorrichtung und/oder eingerichtet, programmiert und/oder konstruiert zum Durchführen eines vorab beschriebenen Verfahrens gelöst. Es ergeben sich die vorab beschriebenen Vorteile.

Weitere Vorteile ergeben sich aus den Unteransprüchen und der nachfolgenden Beschreibung eines bevorzugten Ausführungsbeispiels.

### Kurzbeschreibung der Figuren

Im Folgenden wird die Erfindung anhand eines Ausführungsbeispiels näher erläutert. Es zeigen:
- Fig. 1: ein Blockschaltbild eines Gerätesets mit einer Auslesevorrichtung und einer mit der Auslesevorrichtung zusammenwirkenden Messanordnung zum Messen einer Luftfeuchtigkeit an einem Messort;
- Fig. 2: ein Ablaufdiagramm eines Verfahrens zum Messen einer Luftfeuchtigkeit an einem Messort; und
- Fig. 3: Verläufe einer Messgröße und einer aus der Messgröße ermittelbaren Feuchtigkeitsgröße.

### Beschreibung bevorzugter Ausführungsbeispiele

Die Erfindung wird jetzt beispielhaft unter Bezug auf die beigefügten Zeichnungen näher erläutert. Allerdings handelt es sich bei den Ausführungsbeispielen nur um Beispiele, die nicht das erfinderische Konzept auf eine bestimmte Anordnung beschränken sollen. Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

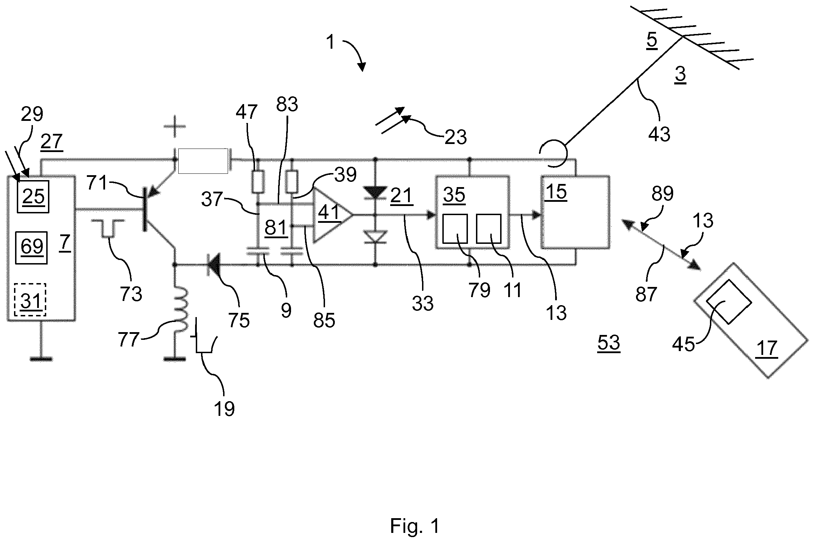

Fig. 1 zeigt ein Blockschaltbild einer Messanordnung zum Messen einer Luftfeuchtigkeit 3 an einem Messort 5. Die Messanordnung 1 weist eine elektrische Energiequelle 7 auf. Die elektrische Energiequelle 7 dient vorzugsweise zum energieautarken und/oder netzunabhängigen versorgen der Messanordnung 1 mit elektrischer Energie. Dazu weist die elektrische Energiequelle 7 einen Wandler 25, eine Impulserzeugungsvorrichtung 69 und einen Energiespeicher 31 auf. Mittels des Wandlers 25 kann eine in einer Umgebung 27 der Messanordnung 1 vorhandene Umgebungsenergie 29 in die elektrische Energie umgewandelt werden. Der Wandler 25 weist insbesondere eine Fotozelle und/oder einen thermoelektrischen Wandler auf.

Die mittels des Wandlers 25 erzeugbare elektrische Energie kann in dem Energiespeicher 31 zwischengespeichert werden. Die so kontinuierlich zur Verfügung stehende zwischengespeicherte elektrische Energie kann mittels der Pulserzeugungsvorrichtung 69 in in bestimmten Zeitabständen zur Verfügung stehende Versorgungspulse 73 umgewandelt werden. Optional weist der Energiespeicher 31 einen Kondensator zum Puffern und/oder eine austauschbare Batterie auf.

Bevorzugt versorgt jedoch die elektrische Energiequelle 7 die Messanordnung 1 energieautark und/oder netzunabhängig mit der elektrischen Energie. Dies bedeutet, dass die Messanordnung 1 weder eine zusätzliche Batterie benötigt noch an ein elektrisches Stromnetz angeschlossen werden muss, um betrieben zu werden. Die Messanordnung 1 ist also batteriefrei und netzunabhängig betreibbar. Bei der Alternative mit der Batterie versorgt diese bzw. ist diese so dimensioniert, dass die Messanordnung 1 für einen Zeitraum > 1 Jahr, insbesondere >2 Jahre, insbesondere > 5 Jahre, insbesondere > 10 Jahre mit der elektrischen Energie unterbrechungsfrei versorgbar ist. Vorteilhaft ist die Messanordnung 1 zwischen den elektrischen Pulsen im Wesentlichen stromlos. Im Wesentlichen stromlos kann vorliegend bedeuten, dass mit Ausnahme eines von der Pulsgeneratorvorrichtung 69 verursachten Stromflusses zwischen den Pulsen in der Messanordnung 1 keine weiteren Ströme fließen.

Das elektrische Schaltelement 71, das insbesondere als Transistor ausgebildet ist, wird an einer Basis mit den Versorgungspulsen 73 angesteuert. Solange also der Versorgungspuls 73, der die elektrische Energie von der elektrischen Energiequelle 7 liefert anliegt, ist das elektrische Schaltelement 71 durchgeschaltet. In diesem Zustand sperrt eine der übrigen Messanordnungen 1 vorgeschaltete Diode 75 und eine Spule 77 wird bestromt. Die Spule 77 baut dabei ein Magnetfeld auf, das eine gewisse Menge der elektrischen Energie aus dem Energiespeicher 31 zwischenspeichert. Sobald der Versorgungspuls 73 zu Ende ist, öffnet das elektrische Schaltelement 71 und die Spule 77 wird nicht mehr bestromt. Dadurch wird die im elektrischen Feld gespeicherte elektrische Energie in Form eines weiteren elektrischen Pulses 19 frei. Mittels des weiteren elektrischen Pulses 19 wird die Messanordnung 1 zum Durchführen einer Messung der Luftfeuchtigkeit 33 kurzzeitig bestromt. Die Spule 77 dient also zum Bereitstellen der elektrischen Energie für die Messanordnung 1, wobei in diesem Zustand also während des weiteren elektrischen Pulses 19 die Diode 75 durchgeschaltet ist. Dies bietet den Vorteil, dass die von der elektrischen Energiequelle 7 gelieferte Ausgangsspannung Schwankungen unterliegen kann, beispielsweise verursacht durch unterschiedliche Ladezustände des Energiespeichers 31. Der Energiespeicher 31 kann als Kondensator zum Puffern der von dem Wandler 25 erzeugten Energie ausgelegt sein. Durch eine Spannungserhöhung mittels der Spule 77 und der gleichzeitigen Spannungsbegrenzung mittels der Diodenvorrichtung 21 bleibt die Spannung der Vielzahl der weiteren elektrischen Pulse 19 jeweils gleich und es ändert sich lediglich eine Pulsdauer, was für die jeweilige Messung und Erzeugung des Signals 23 unkritisch ist.

Der elektrischen Energiequelle 7 ist die Diode 75 und dieser eine Messvorrichtung 81 zum Messen der Luftfeuchtigkeit 3 nachgeschaltet. Die Messvorrichtung 81 wird mittels einer Vielzahl der weiteren elektrischen Pulse 19 mit der elektrischen Energie versorgt. Die Diode 75 lässt jeweils einen negativen Anteil der weiteren elektrischen Pulse 19 durch. Die Messvorrichtung 81 weist eine Messstrecke 37 und dieser parallel geschaltet eine Referenzstrecke 39 auf. In die Messstrecke 37 ist eine Sensorvorrichtung 9 geschaltet. Die Sensorvorrichtung 9 ist beispielhaft als Kondensator ausgebildet, der abhängig von der an dem Messort 5 herrschenden Luftfeuchtigkeit 3 unterschiedliche Kapazitäten ausweist, ausgebildet. Außerdem ist in die Messstrecke 37 ein Messwiderstand geschaltet. Der Messwiderstand ist in Serie zu der Sensorvorrichtung 9 geschaltet. Dadurch weist die Messstrecke 37 eine Messbrücke auf, die zwischen dem Messwiderstand und der Sensorvorrichtung 9 einen Messausgang 83 aufweist. Analog dazu ist die Referenzstrecke 39 aufgebaut und weist dementsprechend eine Referenzkapazität und einen Referenzwiderstand auf, die zusammen eine Referenzbrücke mit einem Referenzausgang 85 bilden. Bevorzugt sind der Referenzwiderstand und der Messwiderstand gleich groß. Außerdem ist bevorzugt die Referenzkapazität an eine Kapazität der Sensorvorrichtung 9 bei einer vorgegebenen und/oder vorgebbaren Luftfeuchtigkeit 3 angepasst. Sofern die vorgegebene Luftfeuchtigkeit 3 herrscht, liefern also die Messstrecke 37 und die Referenzstrecke 39 am Messausgang und am Referenzausgang 85 identische Spannungen. Bevorzugt ist es denkbar, anstelle des Messwiderstands und des Referenzwiderstands ebenfalls Kapazitäten vorzusehen. Dadurch ergeben sich zwei entsprechend aufgebaute parallel geschaltete rein kapazitive Brücken, die beim Einschalten des weiteren elektrischen Pulses dem Komparator 41 schneller entsprechende Ausgangsspannungen liefern. Der Messausgang 83 und der Referenzausgang 85 weisen dadurch ein besseres Zeitverhalten auf. Die kapazitive Ausgestaltung der entsprechend beschalteten Messbrücke und der Referenzbrücke wird in Verbindung mit der gepulsten Bestromung mit den weiteren elektrischen Pulsen 19 möglich.

Der Messausgang 83 der Messstrecke 37 und der Referenzausgang 85 der Referenzstrecke 39 sind einem Komparator 41 vorgeschaltet. Der Komparator 41 verarbeitet also die Signale des Messausgangs 83 und des Referenzausgangs 85 und vergleicht diese miteinander. Je nachdem welche Spannung höher liegt, liefert der Komparator 41 unterschiedliche Ausgangssignale. Vorzugsweise liefert er eine Schwellwertgröße 33, die beispielsweise einen Wert von 0 Volt und einen Wert der Versorgungsspannung, also des weiteren elektrischen Pulses 19 annehmen kann. Alternativ sind auch andere Spannungswerte der Schwellwertgröße 33 möglich. Insbesondere handelt es sich bei der Schwellwertgröße 33 um eine binäre Größe, die die Messgröße der Messanordnung 1 darstellt.

Am Ausgang des Komparators 41, also diesem nachgeschaltet, weist die Messanordnung 1 eine Diodenvorrichtung 21 auf. Je nach Wert der Schwellwertgröße 33 also des Ausgangs des Komparators 41 leuchtet entweder eine erste Leuchtdiode oder eine zweite Leuchtdiode der Diodenvorrichtung 21. Da der Ausgang des Komparators 41 die Messgröße liefert und von der Kapazität der Sensorvorrichtung 9 abhängt, leuchtet eine der beiden Leuchtdioden der Diodenvorrichtung 21 abhängig davon, ob die an dem Messort 5 herrschende Luftfeuchtigkeit 3 oberhalb einer Schwelle liegt oder diese Schwelle unterschreitet.

Dem Komparator 41 ist neben den zwei Leuchtdioden der Diodenvorrichtung 21 außerdem eine Datenerfassung 35 nachgeschaltet. Die Datenerfassung 35 wird vorzugsweise durch den weiteren elektrischen Puls 19 aufgeweckt und ermittelt und/oder berechnet einen in den stromlosen Phasen zwischengespeicherten Wert der Feuchtigkeitsgröße 13. Das Ermitteln und/oder Berechnen erfolgt vorzugsweise abhängig von der Schwellwertgröße 33.

Als Ausgang liefert die Datenerfassung 35 eine Feuchtigkeitsgröße 13, die von den pro elektrischem Puls 19, also zyklisch erzeugten einzelnen Messgrößen, insbesondere in Form der Schwellwertgröße 33, abhängt. Die Feuchtigkeitsgröße 13 ist damit eine Bewertungsgröße, die dazu geeignet ist die an dem Messort 5 über einen längeren Zeitraum anzutreffende Luftfeuchtigkeit 3 zu bewerten.

Insbesondre ist es möglich, mittels der Feuchtigkeitsgröße 13 darauf zu schließen, ob die Luftfeuchtigkeit 3 überwiegend in einem kritischen Bereich, beispielsweise bei der sich Schimmel bildet, oder in einem unkritischen Bereich, bei der eine Schimmelbildung nicht zu erwarten ist, liegt. Vorteilhaft kann die Feuchtigkeitsgröße 13 also nach einem längeren Messzeitraum, beispielsweise > 1 Jahr abgerufen werden und anhand dieser die Bewertung kritisch oder unkritisch getroffen werden.

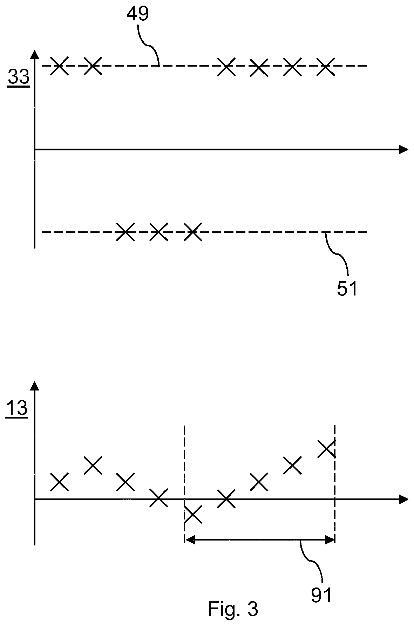

Gemäß einer Alternative ist es denkbar, dass zumindest ein in Fig. 3 eingezeichnetes Intervall 91 gebildet wird, das Teil der Langzeitmessung ist. Das Intervall 91 ist beispielsweise zeitlich so bemessen, dass falls die Feuchtigkeitsgröße 3 einen weiteren Schwellwert überschreitet, mit hoher Wahrscheinlichkeit eine Schimmelbildung zu erwarten ist. Insbesondere kann die Langzeitmessung in eine Vielzahl solcher Intervalle unterteilt sein, wobei pro Intervall eine eigene Feuchtigkeitsgröße 3 berechnet, gespeichert, bereitgestellt und/oder an die Auslesevorrichtung 17 übermittelt wird.

Der Datenerfassung 35 ist eine Schnittstellenvorrichtung 15 nachgeschaltet. Eine Speichervorrichtung 11 dient zum Zwischenspeichern der jeweils mittels der Datenerfassung 35 ermittelten und/oder erfassten Feuchtigkeitsgröße 13. Die Speichervorrichtung 11 ist beispielsweise in der Datenerfassung 35 vorgesehen. Die Feuchtigkeitsgröße 13 wird also mittels der Speichervorrichtung 11 und/oder der Schnittstellenvorrichtung 15 während der gesamten Messdauer bereitgehalten. Für den Fall, dass diese benötigt wird, kann die Feuchtigkeitsgröße 13 ausgelesen werden. Dies erfolgt bevorzugt mittels einer Auslesevorrichtung 17 die eine Datenstrecke 87 zwischen der Ausleseschnittstellenvorrichtung 17 und der Messanordnung 1, insbesondere der Schnittstellenvorrichtung 15 der Messanordnung 1, etabliert. Dazu weist die Auslesevorrichtung 17 eine Auslesevorrichtung 45 auf. Mittels des Auslesens kann die Feuchtigkeitsgröße 13 über die Datenstrecke 87 auf die Auslesevorrichtung 17 übertragen werden. Insbesondere kann diese dort zwischengespeichert, angezeigt und/oder weitervermittelt werden. Optional weist die Datenerfassung 35 eine Echtzeituhr 79 auf. Die Echtzeituhr 79 kann über das Zeit-Funksignal stets die genaue Uhrzeit liefern. Mittels der Uhrzeit kann die Feuchtigkeitsgröße 13 einen oder mehrere Zeitstempel erhalten. Ferner ist es denkbar eine Vielzahl von jeweils zeitlich einordenbaren Feuchtigkeitsgrößen 13 zu ermitteln, um eine noch bessere zeitliche Bewertung des Raumklimas zu ermöglichen. Ferner können dadurch auch Rückschlüsse auf ein Verhalten eines Nutzers des Raumes und/oder der Messanordnung gezogen werden, beispielsweise über ein Lüftungsverhalten des Nutzers.

Bevorzugt ist die Diodenvorrichtung 21 so ausgelegt, dass diese ein Signal 23, das von der an dem Messort 5 gemessenen Feuchtigkeit 3 abhängt, wiedergeben kann. Beispielsweise kann ein erstes Blitzsignal wiedergegeben werden, falls sich die Luftfeuchtigkeit 3 in einem unkritischen Bereich und ein zweites Blitzsignal, falls sich die Luftfeuchtigkeit in einem kritischen Bereich befindet. Falls das zweite Blitzsignal wiedergeben wird, kann dies beispielsweise signalisieren, dass der Raum gelüftet werden muss, also Maßnahmen zum Reduzieren der Luftfeuchtigkeit 3 zu ergreifen sind. Das Signalisieren kann mittels unterschiedlicher Farben erfolgen. Beispielsweise rot für kritisch wenn der Raum gelüftet werden muss und grün wenn genug gelüftet ist beziehungsweise keine Maßnahme zum Reduzieren der Luftfeuchtigkeit 3 erforderlich ist.

Gemäß einer weiteren Alternative, weist die Diodenvorrichtung 21 lediglich normale Dioden auf, vorzugsweise nur eine Diode, beispielsweise eine Zenerdiode. Diese dient/dienen dann zum Regeln/Begrenzen der Spannung des jeweiligen weiteren elektrischen Pulses 19. Falls die Diodenvorrichtung 21 jedoch Leuchtdioden aufweist, erfüllt diese zwei Funktionen, nämlich das Wiedergeben des Signals 23 und gleichzeitig ein Spannungsbegrenzen des elektrischen Pulses 19. Sobald das Schaltelement 71 sperrt, erzeugt die Spule 77 in Form eines Rückschlagpulses, also des elektrischen Pulses 19 eine vergleichsweise hohe Spannung, die dann entsprechend abfällt. Um für die Messung und/oder den Betrieb der Datenerfassung 35 und/oder der Schnittstellenvorrichtung 15 eine gleichbleibende Spannung zur Verfügung zu haben, wird mittels der Diodenvorrichtung 21 der elektrische Puls 19 spannungsbegrenzt. Dies erfolgt aufgrund der stark nicht linearen Kennlinien der Dioden der Diodenvorrichtung 21. Dadurch kann eine besonders genaue Messung mittels der Messstrecke 37 bzw. der Messvorrichtung 81 erfolgen, wobei gleichzeitig die übrigen Komponenten, also die Datenerfassung 35, der Komparator 41 und die Schnittstellenvorrichtung 15 vor Überspannungen geschützt sind.

Alternativ oder zusätzlich kann die Datenerfassung 35 in Form eines Mikrocontrollers ausgeführt sein, der pro elektrischem Puls 19 aufgeweckt wird und eine entsprechende Verarbeitung der Messgröße, also der Schwellwertgröße 33 vornimmt. Alternativ oder zusätzlich können die Schnittstellenvorrichtung 15 und die Datenerfassung 35 in einem Bauteil integriert werden. Alternativ oder zusätzlich kann die Messgröße auf abweichende Art verarbeitet werden. Dazu kann beispielsweise ein Mikrokontroller eingesetzt werden, der entsprechende Rechenoperationen vornimmt. Beispielsweise kann anstelle des Komparators 41 ein mehr als nur zwei Stufen aufweisender Analog-Digital-Wandler (AD) eingesetzt werden, dem der Mikrokontroller nachgeschaltet ist. Dieser kann die Anzeige, die Verarbeitung, Speicherung und/oder die Übermittlung bewerkstelligen. Der Mikrokontroller kann einen dem AD-Wandler nachgeschalteten Integrator aufweisen.

Zum Schutz der Messanordnung 1 vor Manipulationen kann diese mittels einer Manipulationsschutzvorrichtung 43, beispielsweise einer Plombe an dem Messort 5 fixiert und/oder manipulationsgesichert werden.

Gemäß einer weiteren Alternative kann die Messanordnung 1 eine Temperaturkompensation 47 aufweisen. Die Temperaturkompensation 47 kann entweder in die Messstrecke 37 und/oder die Referenzstrecke 39 geschaltet sein. Beispielsweise kann der Messwiderstand der Messstrecke 37 als temperaturempfindlicher Widerstand ausgebildet sein. Der temperaturempfindliche Widerstand kann beispielsweise als NTC oder PTC Widerstand ausgebildet sein. Dadurch kann die Messgröße bzw. die jeweilige Schwellwertgröße 33 und damit die daraus errechnete Feuchtigkeitsgröße 13 zusätzlich zu der Luftfeuchtigkeit 3 auch von einer Temperatur an dem Messort 5 abhängig gemacht werden. Beispielsweise kann dadurch auch ein unterschiedliches Verhalten von Schimmelbildung je nach Temperatur und/oder der Umstand, dass die Feuchtigkeitsaufnahmekapazität von Luft von der Temperatur abhängt, Rechnung getragen werden.

Die Messanordnung 1 und die Auslesevorrichtung 17 bilden ein Geräteset zum Messen und/oder Bewerten der Luftfeuchtigkeit 3 an dem Messort 5. Insbesondere kann eine Vielzahl von Messanordnungen 1 an unterschiedlichen Messorten 5 aufgestellt werden, die nach Ablauf eines Messzeitraumes mittels nur einer Auslesevorrichtung 17 nacheinander ausgelesen werden können. Alternativ ist es auch denkbar, dass die Auslesevorrichtung 17 gleichzeitig mehrere der Datenstrecken 87 aufbaut, sodass die Vielzahl der Messanordnungen 1 gleichzeitig ausgelesen werden kann.

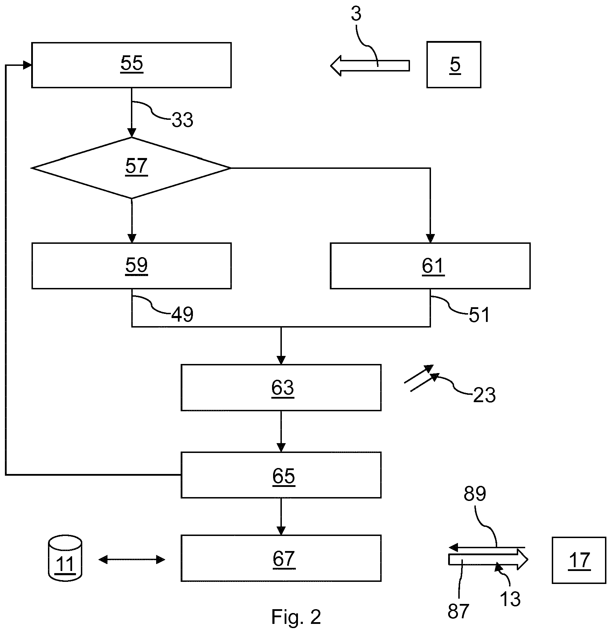

Fig. 2 zeigt ein Ablaufdiagramm eines Verfahrens zum Messen, speichern und/oder auslesen einer der Luftfeuchtigkeit 3 an dem Messort 5. Das Verfahren kann insbesondere mittels der in Fig. 1 gezeigten Messanordnung 1 durchgeführt werden. Insofern wird auch auf die Beschreibung der Fig. 1 verwiesen. In einem ersten Schritt 55 erfolgt das Messen der Luftfeuchtigkeit 3. Dazu wird vorzugsweise mittels des Komparators 41 die Messgröße, also die Schwellwertgröße 33 ermittelt. Die Schwellwertgröße 33 wird einem Vergleich 57 zugeführt. Mittels des Vergleichs 57 wird entschieden, ob die Luftfeuchtigkeit 3 oberhalb eines Schwellwertes oder unterhalb eines Schwellwertes liegt. Für den Fall, dass die Luftfeuchtigkeit oberhalb des Schwellwertes liegt, verzweigt der Vergleich 57 in einen zweiten Schritt 59 zum Setzen eines ersten Wertes 49. Für den Fall, dass die Luftfeuchtigkeit 3 unterhalb des Schwellwertes liegt, verzweigt der Vergleich 57 in einen dritten Schritt 61, wobei ein zweiter Wert 51 gesetzt wird. Die Schwellwertgröße 33 nimmt also entweder den ersten Wert 49 oder den zweiten Wert 51 an. Abhängig von der Schwellwertgröße 33, also den Werten 49 und 51 wird in einem vierten Schritt 63 das Signal 23 wiedergeben, also angezeigt ob die Luftfeuchtigkeit 3 sich in einem kritischen Bereich befindet oder nicht. Der vierte Schritt 63 des Anzeigens ist optional.

In einem nachfolgenden fünften Schritt 65 wird die Feuchtigkeitsgröße 13 berechnet bzw. ermittelt, insbesondere mittels der Datenerfassung 35. Die Datenerfassung kann einen Mikroprozessor aufweisen. Der fünfte Schritt 65 mündet zyklisch in den ersten Schritt 55, sodass das Verfahren von neuem beginnt, also die Luftfeuchtigkeit 3 an dem Messort 5 erneut gemessen wird. Dies fällt zusammen mit der Erzeugung des elektrischen Pulses 19. Das Verfahren wird also im Takt der Versorgungspulse 73 der elektrischen Energiequelle durchgeführt. Außerdem verzweigt der fünfte Schritt 65 in einen sechsten Schritt 67 bei dem die Feuchtigkeitsgröße 13 bereitgestellt wird. Insbesondere wird dazu die Feuchtigkeitsgröße 13 in der Speichervorrichtung 11, gespeichert und so für ein Auslesen der Feuchtigkeitsgröße 13 mittels der Auslesevorrichtung 17 bereitgehalten. Das Auslesen erfolgt über die Datenstrecke 87. Zum Etablieren der Datenstrecke 87 ist es möglich, dass die Auslesevorrichtung 17 ein Steuersignal an die Schnittstellenvorrichtung 15 an die Messvorrichtung 1 sendet, insbesondere zum Aufwecken und Etablieren der Datenstrecke 87. Die Datenstrecke 87 kann über Funk und/oder optisch aufgebaut werden, beispielsweise über ein Opto-Id-Verfahren, bei dem eine optische bidirektionale Kommunikation über die Datenstrecke 87 läuft. Außerdem ist eine Datenübertragung mittels RFID-Technik (Radio-Frequenz-Identifikation) möglich. Insbesondere kann die Messvorrichtung dabei mittels einer Kennung identifiziert werden und/oder die Kommunikation abhörsicher gestaltet werden. Die Datenstrecke kann optisch über eine Distanz von vorzugsweise bis zu 5 m, insbesondere bis zu 10 m aufgebaut werden. Es sind auch kleinere oder größere Distanzen möglich, größere insbesondere über Funk, insbesondere über eine Gebäudehülle hinweg, um einen zu vermessenden Raum nicht betreten zu müssen. Eine Alternative des Verfahrens sieht ein Bereitstellen der Messgröße und/oder der Feuchtigkeitsgröße 13 zur Anzeige vor, wobei insbesondere nur die Anzeige erfolgt, insbesondere als das und/oder mittels des Signal/s 23.

Fig. 3 zeigt zwei Schaubilder jeweils über der Zeit, wobei jeweils auf der x-Achse die Zeit aufgetragen ist, der Schwellwertgröße 33 in einem oberen Schaubild und der Feuchtigkeitsgröße 13 in einem unteren Schaubild. Im oberen Schaubild der Fig. 3 ist zu erkennen, dass die Schwellwertgröße 33, also die Messgröße am Ausgang des Komparators 41 den ersten Wert 49 und den zweiten Wert 51 annehmen kann. Vorliegend handelt es sich beispielhaft um die Werte 1 und -1. Durch ein einfaches Addieren pro elektrischem Puls 19, also pro Zyklus kann die Schwellwertgröße 33 binär integriert werden, was in dem unteren Schaubild der Fig. 3 verdeutlicht ist. Dadurch kann pro Zyklus, also pro elektrischem Puls 19 die Feuchtigkeitsgröße 13 gebildet bzw. aus einer in einem Zyklus n - 1 gespeicherten Feuchtigkeitsgröße 13 fortgeschrieben werden.

Gemäß einem weiteren Ausführungsbespiel ist es möglich, dass die Datenstrecke 87 zu einem Netzwerk aufgebaut wird. Dies kann verschlüsselt und/oder in zeitlichen Abständen erfolgen. Die Feuchtigkeitsgröße 13 kann über das Netzwerk weitergeleitet, verarbeitet und/oder signalisiert werden. Insbesondere kann das Netzwerk Teil einer intelligenten Haussteuerung sein und/oder an eine solche angebunden sein. Bei dem Netzwerk kann es sich um ein Internet handeln.

Darüber hinaus ist es denkbar, dass die Messvorrichtung 81 einen integrierten Schaltkreis aufweist, der digitale Informationen liefert, beispielsweise Temperaturwerte und/oder Feuchtigkeitswerte und/oder die Messgröße 33. Optional kann der integrierte Schaltkreis die Strecken 81, 83 und/oder den Komparator 41 ersetzen oder in integrierter Form aufweisen.

Zusammenfassend ist die Messanordnung 1 bevorzugt energieautark und/oder netzunabhängig, kann eine Langzeitmessung durchführen, über größere Entfernungen auf optischem Wege ausgelesen werden und/oder gleichzeitig eine direkte Signalisierung der aktuellen Luftfeuchtigkeit 3 mittels des Signals 23 vornehmen. Ferner kann das Auslesen alternativ oder zusätzlich über ein Netzwerk erfolgen, vorzugsweise über eine Funkstrecke.

**Bezugszeichenliste**

| | | | |
|---|---|---|---|
| 1 | Messanordnung | 47 | Temperaturkompensation |
| 3 | Luftfeuchtigkeit | 49 | erster Wert |
| 5 | Messort | 51 | zweiter Wert |
| 7 | elektrische Energiequelle | 53 | Geräteset |
| 9 | Sensorvorrichtung | 55 | erster Schritt |
| 11 | Speichervorrichtung | 57 | Vergleich |
| 13 | Feuchtigkeitsgröße | 59 | zweiter Schritt |
| 15 | Schnittstellenvorrichtung | 61 | dritter Schritt |
| 17 | Auslesevorrichtung | 63 | vierter Schritt |
| 19 | elektrischer Puls | 65 | fünfter Schritt |
| 21 | Diodenvorrichtung | 67 | sechster Schritt |
| 23 | Signal | 69 | Pulsgeneratorvorrichtung |
| 25 | Wandler | 71 | Schallelement |
| 27 | Umgebung | 73 | Versorgungspuls |
| 29 | Umgebungsenergie | 75 | Diode |
| 31 | Energiespeicher | 77 | Spule |
| 33 | Schwellwertgröße | 79 | Echtzeituhr |
| 35 | Datenerfassung | 81 | Messvorrichtung |
| 37 | Messstrecke | 83 | Messausgang |
| 39 | Referenzstrecke | 85 | Referenzausgang |
| 41 | Komparator | 87 | Datenstrecke |
| 43 | Manipulationsschutzvorrichtung | 89 | Steuersignal |
| 45 | Ausleseschnittstellenvorrichtung | 91 | Intervall |

## Patentansprüche

1. Messanordnung (1) zum Messen einer Luftfeuchtigkeit (3) an einem Messort (5), mit:
- einer elektrischen Energiequelle (7), mittels der die Messanordnung (1) mit elektrischer Energie versorgbar ist,
- einer Sensorvorrichtung (9), mittels der die Luftfeuchtigkeit (3) messbar ist,
- einer der Sensorvorrichtung (9) nachgeschalteten Speichervorrichtung (11), mittels der eine von der gemessenen Luftfeuchtigkeit (3) abhängige Feuchtigkeitsgröße (13) speicherbar ist,
**gekennzeichnet durch** eine der Speichervorrichtung (11) nachgeschaltete Schnittstellenvorrichtung (15), mittels der die Feuchtigkeitsgröße (13) an eine von der Messanordnung (1) unabhängige Auslesevorrichtung (17) übermittelbar ist.

2. Messanordnung nach Anspruch 1, **dadurch gekennzeichnet, dass** mittels der elektrischen Energiequelle (7) elektrische Pulse (19) erzeugbar sind, wobei die Messanordnung (1) eine Diodenvorrichtung (21) aufweist, mittels der die elektrischen Pulse (19) spannungsbegrenzbar sind und dabei gleichzeitig ein von der Luftfeuchtigkeit (3) abhängiges vorzugsweise optisches Signal anzeigbar ist.

3. Messanordnung nach Anspruch 1 oder 2, **dadurch gekennzeichnet, dass** die elektrische Energiequelle (7) einen Wandler (25) zum Wandeln einer in einer Umgebung (27) der Messanordnung (1) vorhandenen Umgebungsenergie (29) in elektrische Energie aufweist, mittels dem die Messanordnung (1) energieautark und/oder netzunabhängig mit elektrischer Energie versorgbar ist und/oder einen Energiespeicher (31) aufweist, mittels der die Messanordnung (1) für einen Zeitraum von >1 Jahr, >2 Jahre, >5 Jahre, >10 Jahre mit elektrischer Energie versorgbar ist.

4. Messanordnung nach einem der vorhergehenden Ansprüche, **dadurch gekennzeichnet, dass** mittels der Messanordnung (1) eine von der Luftfeuchtigkeit (3) abhängige binäre Schwellwertgröße (33) erzeugbar ist.

5. Messanordnung nach einem der vorhergehenden Ansprüche, **dadurch gekennzeichnet, dass** die Speichervorrichtung (11) Teil einer Datenerfassung (35) ist, die abhängig von der Luftfeuchtigkeit (3) und/oder Temperatur zyklisch die Feuchtigkeitsgröße (13) berechnet, ermittelt und/oder erfasst.

6. Messanordnung nach einem der vorhergehenden Ansprüche, **dadurch gekennzeichnet, dass** die Messanordnung (1) eine von der Luftfeuchtigkeit (3) des Messorts (5) beeinflussbare Messstrecke (37) und eine definierte oder justierbare Eigenschaften aufweisende Referenzstrecke (39) aufweist, denen ein Komparator (41) nachgeschaltet ist.

7. Messanordnung nach Anspruch 6, **dadurch gekennzeichnet, dass** die Messstrecke (37) und/oder die Referenzstrecke (39) eine Temperaturkompensation (47) aufweist und/oder einen integrierten Schaltkreis zur Ermittlung der Luftfeuchtigkeit (3) und/oder einer Temperatur an dem Messort (5) aufweist.

8. Messanordnung nach einem der vorhergehenden Ansprüche, **dadurch gekennzeichnet, dass** eine Manipulationsschutzvorrichtung (43), mittels der die Messanordnung (1) ortsfest an dem Messort (5) fixierbar, verschließbar und/oder zumindest ein Entfernen von dem Messort (5) und/oder ein Öffnen anzeigbar sind.

9. Auslesevorrichtung (17) zum Auslesen einer in einer Messanordnung (1) nach einem der Ansprüche 1 bis 8 gespeicherten Feuchtigkeitsgröße (13), mit einer Ausleseschnittstellenvorrichtung (13), die zum Auslesen der Feuchtigkeitsgröße (13) mit der Schnittstellenvorrichtung (15) der Messanordnung (1) zusammenwirkt.

10. Verfahren zum Messen, Speichern und/oder Auslesen einer Luftfeuchtigkeit (3) an einem Messort (5) mittels einer Messanordnung (1), insbesondere einer Messanordnung (1) nach einem der Ansprüche 1 bis 7, mit:
- Messen der Luftfeuchtigkeit (3) an dem Messort (5) für eine Vielzahl einzelner Zeitpunkte,
- Ermitteln für jeden der Zeitpunkte jeweils eine von der jeweils gemessenen Luftfeuchtigkeit (3) abhängige Messgröße,
- Ermitteln einer von den einzelnen Messgrößen abhängigen Feuchtigkeitsgröße (13),
- Bereitstellen der Feuchtigkeitsgröße (13).

11. Verfahren nach Anspruch 10, **gekennzeichnet durch**:
- Ermitteln der Messgrößen jeweils als binäre Schwellwertgröße (33), die einen ersten Wert (49) und einen zweiten Wert (51) annehmen kann,
- Ermitteln der Feuchtigkeitsgröße (13) jeweils durch Erhöhen und/oder Erniedrigen in Abhängigkeit des ersten Wertes (49) und/oder des zweiten Wertes (51).

12. Verfahren nach einem der Ansprüche 10 oder 11, **gekennzeichnet durch**:
- Auslesen der Feuchtigkeitsgröße (13) mittels der Auslesevorrichtung (17).

13. Verfahren nach einem der Ansprüche 10 bis 12, **gekennzeichnet durch**:
- Manipulationssicheres und/oder manipulationsanzeigendes Verschließen und/oder Fixieren der Messanordnung an dem Messort (5) während des Messens der Luftfeuchtigkeit (3) und/oder
- Bereitstellen der Feuchtigkeitsgröße (13) für eine Übermittlung an eine von der Messanordnung (1) unabhängige Auslesevorrichtung (17).

14. Verfahren nach einem der Ansprüche 10 bis 13, **gekennzeichnet durch**:
- Erzeugen eines elektrischen Pulses (19),
- Anzeigen der Messgröße,
- Spannungsbegrenzen des elektrischen Pulses (19) während und durch das Anzeigen der Messgröße,
- Ermitteln der Messgröße während und unter Verwendung der elektrischen Energie des spannungsbegrenzten elektrischen Pulses (19).

15. Geräteset (53) mit einer Messanordnung (1) nach einem der Ansprüche 1 bis 7 und einer Auslesevorrichtung (17) nach Anspruch 8 und/oder eingerichtet, konstruiert und/oder programmiert zum Durchführen eines Verfahrens nach einem der Ansprüche 10 bis 14.
